# EUROPÄISCHE PATENTSCHRIFT

(11) **EP 2 916 797 B1**
(45) Veröffentlichungstag und Bekanntmachung des Hinweises auf die Patenterteilung: **08.07.2020**
(21) Anmeldenummer: 13838085.2
(22) Anmeldetag: 08.11.2013
(51) Int. Cl.: A61H 31/00

(54) **VORRICHTUNG ZUR KONTROLLIERTEN HERZ-LUNGEN-REANIMATION BEI HERZSTILLSTAND**
DEVICE FOR A CONTROLLED HEART-LUNG RESUSCITATION IN THE EVENT OF A CARDIAC ARREST
DISPOSITIF PERMETTANT UNE RÉANIMATION CARDIO-PULMONAIRE CONTRÔLÉE EN CAS D'ARRÊT CARDIAQUE

(30) Priorität: 08.11.2012 DE 102012021828; 11.02.2013 DE 102013002428; 15.04.2013 DE 102013006469
(43) Veröffentlichungstag der Anmeldung: 16.09.2015
(73) Patentinhaber: Mössmer, Sebastian, 81377 München (DE); Gleixner, Josef, 92507 Nabburg (DE)
(72) Erfinder: MÖSSMER, Sebastian, 81687 München (DE); HAGL, Christian, 81377 München (DE); RÖHLK, Kathrin, 80637 München (DE); GLEIXNER, Josef, 92507 Nabburg (DE)
(74) Vertreter: Szaunig, Bernd
(86) Internationale Anmeldenummer: PCT/DE2013/000665
(87) Internationale Veröffentlichungsnummer: WO 2014/071915

(56) Entgegenhaltungen:
- WO-A1-2012/143000
- US-A- 4 554 910

## Beschreibung

Die vorliegende Erfindung befasst sich mit einer Vorrichtung zur kontrollierten Herz-Lungen-Reanimation bei Herzstillstand mit einem deutlich hörbaren Signal beim Erreichen eines Grenzdruckes, insbesondere mit einer Vorrichtung, mit einem speziellen Feder- und Formsystem, das die Handhabung bei Anwendung der Vorrichtung erleichtert.

Derartige Vorrichtungen sind im Stand der Technik aus der US 4,554,910. bekannt. Aus dieser Druckschrift ist eine Vorrichtung nach dem Oberbegriff von Anspruch 1 bekannt.

Diese Druckschrift offenbart eine Vorrichtung zur Herz-Lungen-Reanimation bei Herzstillstand mit mindestens einem Druckübertragungsmittel und mindestens einem Druck aufnehmenden Element und einer Druckanzeige, die bei Eintritt eines mechanischen Grenzdruckes (Fₘₐₓ) ein durch menschliche Sinnesorgane wahrnehmbares Signal (S) erzeugt. Zwischen dem mindestens einen Druckübertragungsmittel und dem mindestens einen Druck aufnehmenden Element ist ein Federsystem mit zwei unterschiedlichen Federn angeordnet, das bei Erreichen eines vorbestimmten Grenzdruckes durch eines der beiden Federn ein hörbares erstes Click-Signal erzeugt und bei Nachlassen des Grenzdruckes ein zweites. Das erste und zweite akustische Click-Signal wird mittels einer u-förmigen Blattfeder erzeugt, die etwa in der Mitte der zweiten Feder angeordnet ist und als Wendelfeder ausgebildet ist. Als nachteilig an einem derartigen Gerät zur Herz-Lungen-Reanimation bei Herzstillstand wird es empfunden, dass der mechanische Druck stets zentral auf das erste Druckübertragungsmittel einwirken muss, um die gewünschte Wirkung zur Reanimation zu erzielen. Dies ist im praktischen Einsatz nicht möglich.

Ein ähnliches längliches Gerät ist aus der CN 201304070 Y bekannt geworden, das auch zwei Druckübertragungsmittel aufweist, wischen denen eine Wendelfeder angeordnet ist und bei Erreichen eines Grenzdruckes ein hörbares Signal erzeugt, dass dem Anwender signalisiert, das Druckübertragungsmittel wieder zu entlasten. Als nachteilig an diesem Gerät wird es empfunden, dass es kaum möglich ist, im Notfall eine stabile Position auf dem Brustkorb zu erhalten.

Die Druckschrift WO 2006/101400 A1 offenbart eine Vorrichtung zur manuellen Druckerzeugung auf dem Brustkorb eines menschlichen Körpers. Diese Vorrichtung weist einen mechanischen Tongenerator auf, der bei Erreichen eines vorbestimmten Druckes einen Ton erzeugt. Dazu wird eine Platte in eine Halterung gebracht, die die Platte in einer gekrümmten Vorspannung hält und beim Durchdrücken der Platte einen Ton erzeugt. Die Druckmessung selbst wird mittels eines anderen Mechanismus durchgeführt, der in der WO 2004/056303 A1 näher beschrieben wird. Als nachteilig an einer derartigen Vorrichtung zur Herz-Lungenmassage wird es empfunden, dass infolge des absoluten Funktionssicherheitserfordernisses einer derartigen Vorrichtung das Zusammenwirken aller mechanischen Komponenten zu kompliziert erscheint, so dass die gewünschte Sicherheit nicht gewährleistet werden kann.

Weiterhin ist aus der Druckschrift DE 1491611 ein tragbares Herzmassagegerät bekannt geworden, das aus einer Grundplatte und einem darüber angeordneten Stempel besteht, wobei der Stempel zyklisch mittels einer pneumatischen Mechanik betätigt wird und so auf den Thorax des menschlichen Körpers einwirkt. Da es allgemein bei einem Herzstillstand darauf ankommt, eine Reanimation so schnell wie möglich durchzuführen, sind häufig die zur Verfügung stehenden Geräte in ihrer Handhabung zu aufwendig und kompliziert zu bedienen, so dass dadurch wertvolle Zeit zur Wiederbelebung des menschlichen Körpers verloren gehen kann, was weitreichende Folgen hat.

Darüber hinaus sind Defibrillatoren sind im Stand der Technik wohl bekannt und werden auf Intensivstationen, in Operationssälen, in Notfallaufnahmen, sowie in Fahrzeugen des Rettungsdienstes bereitgehalten und seit den 1990er-Jahren zunehmend auch in öffentlich zugänglichen Gebäuden wie Bahnhöfen, Flughäfen und anderen Orten für eine Anwendung durch medizinische Laien.

Daher ist es Aufgabe der vorliegenden Erfindung, ein einfaches und leicht von Laien auf diesem Gebiet zu bedienendes Gerät zur Herz-Lungen-Reanimation bereitzustellen, das in der Lage ist, einerseits einen kontrollierten sicheren Druck zyklisch auf den Thorax des menschlichen Körpers einwirken zu lassen, der von der Lage des Druckzentrums auf das Gerät weitgehend unabhängig ist.

Diese Aufgabe wird erfindungsgemäß durch die Vorrichtung nach Anspruch 1 gelöst. Weitere erfindungswesentliche Merkmale sind den Unteransprüchen und der Detailbeschreibung zu entnehmen.

Mit der vorliegenden Erfindung wird eine Vorrichtung zur kontrollierten Kardiopulmonalen-Reanimation vorgestellt, die in der Lage ist, eine rasch und unkomplizierte Reanimation eines menschlichen Körpers bei Herzstillstand durchführen zu können. Die geometrischen Ausmaße der erfindungsgemäßen Vorrichtung sind vergleichsweise gering und liegen etwa zwischen 10 und 25 cm im Durchmesser und ca. 6 bis 12 cm in der Höhe. In der Anwendung wird auf ein Druckübertragungsmittel ein mechanischer Druck (F) zyklisch ausgeübt, der bei Erreichen einer maximalen Kraftausübung (Fₘₐₓ) ein mit menschlichen Organen wahrnehmbares Signal erzeugt, was infolge des Zusammenwirkens von Federelementen bewirkt wird, die zwischen dem ersten Druckübertragungsmittel und einer Basisplatte angeordnet sind. In dem Gehäuse der Vorrichtung ist wahlweise ein Defibrillator integriert, der unmittelbar nach der Herz-Lungenmassage angewendet wird.

Die erfindungsgemäße Vorrichtung zur kontrollierten Herz-Lungen-Reanimation des menschlichen Körpers bei Herzstillstand mit mindestens einem Druckübertragungsmittel mit einer Druckanzeige, das zumindest unmittelbar und/oder mittelbar mit dem zu behandelnden Brustkorb an vorbestimmter Stelle angeordnet ist und bei Eintritt eines Grenzdruckes (Fₘₐₓ) ein durch Sinnesorgane wahrnehmbares Signal (S3, S4) erzeugt, ist gekennzeichnet durch mindestens ein flächiges einstückiges Federelement, das bei Einwirkung eines mechanischen Druckes (F) einen einstellbaren Grenzdruck (Fₘₐₓ) erkennt und bei Nachlassen des mechanischen Druckes (Fₘₐₓ) wieder in die ursprüngliche Startposition des Federelements spontan zurückspringt, wobei sowohl beim Erreichen des Grenzdruckes (Fₘₐₓ) als auch beim Zurückspringen in die Startposition ein deutlich hörbares Signal (S) erzeugt, das sowohl mechanisch als auch akustisch auf die das Federelement tragenden Elemente einwirkt. Dieses Druckanzeigegerät umfasst mindestens ein Federsystem mit einer Mehrzahl von Federelementen, die bei Ausübung des mechanischen Druckes (F) zusammenwirken, wobei mindestens ein Federelement flächig und einstückig ausgebildet ist und mindestens zwei Federelemente seitlich vom flächigen einstückigen Federelement und um das flächige einstückige Federelement herum angeordnet sind, wobei oberhalb des mindestens einen flächigen einstückigen Federelements mindestens ein weiteres Federelement des Federsystems angeordnet ist, das den mechanischen Druck auf das mindestens eine Druckübertragungsmittel aufnimmt und auf das flächige einstückige Federelement überträgt.

Von Vorteil für die Vorrichtung ist es ferner, dass die Querschnittsform durch den Mittelpunkt mindestens eines Federelements in etwa trapezförmig ausgebildet ist, wobei g1 die untere Grundlinie und g2 die obere Grundlinie des Trapezes darstellen.

Ein weiterer Vorteil ist darin zu sehen, dass die beiden Seiten S1 und S2 des Trapezes mit der Grundlinie g1 einen spitzen Winkel (α1, α2)) einnehmen, wobei (α1) und (α2) nicht notwendig gleich sind.

Vorteilhaft ist es auch, dass die Wandstärke (d) des flächigen einstückigen Mantels zwischen 0,5 und 1 mm liegt, vorzugsweise bei etwa 0,3 mm, wenn das verwendete Material ein Federstahl ist.

Vorteilhaft ist es ferner, dass die Ränder des Innen- und Außenumfangs Deformationen und Einkerbungen aufweisen, die die Steifigkeit des Federelements beeinflussen und definieren.

Vorteilhaft ist es weiterhin, dass mindestens ein Druckübertragungsmittel ein relativ starres Element ist, das mit mindestens einem weiteren Druckübertragungsmittel direkt oder indirekt in Verbindung steht.

Ein weiterer Vorteil ist darin zu sehen, dass mindestens ein Druckübertragungsmittel mit dem zu behandelnden Körper direkt in Verbindung steht, wobei die Auflagefläche des Druckübertragungsmittels den anatomischen Gegebenheiten angepasst ist.

Vorteilhaft ist es auch, dass die Druckanzeige und/oder Grenzdruckanzeige sowohl elektronisch als auch mechanisch und/oder optisch ausgebildet sein kann.

Vorteilhaft ist es ferner, dass die Signal (S) erzeugende Einheit mindestens mit einem Druck aufnehmenden Element ausgestattet ist.

Ein weiterer Vorteil besteht darin, dass mindestens ein Druckübertragungsmittel rückexpandierend ausgebildet ist.

Vorteilhaft ist es ferner, dass an einer vorbestimmten Stelle, vorzugsweise auf der Unterseite oder im Handbereich auf der Oberseite ein kennzeichnendes Element angeordnet ist, das die richtige Positionierung der Vorrichtung auf dem Brustkorb bestimmt.

Vorteilhaft ist es weiterhin, dass die Vorrichtung eine integrierte Elektronik mit mindestens einem Sensor aufweisen kann, der den mechanischen Druck aufnimmt und über die Elektronik ein Signal (S) erzeugt.

Vorteilhaft ist dabei, dass zumindest mittelbar und/oder unmittelbar die erfindungsgemäße Vorrichtung mit dem zu behandelnden Brustkorb an vorbestimmter Stelle angeordnet wird.

Vorteilhaft ist es darüber hinaus, dass um das mindestens eine flächige einstückige Federelement, mindestens ein weiteres Federelement angeordnet ist, das den mechanischen Druck auf das mindestens eine Druckübertragungsmittel ausgleicht und das Druckübertragungsmittel quasi schwimmend abgefedert ist.

Ferner ist es vorteilhaft, dass die weiteren Federelemente um das erste flächige Federelement angeordnet sind und in einem Ausführungsbeispiel als Spiral- oder Wendelfedern ausgebildet sind.

Das mit dieser Vorrichtung arbeitende Verfahren zur Reanimation der Herz-Lungentätigkeit umfasst folgende Verfahrensschritte:
- P1: Positionieren der Vorrichtung auf dem Brustkorb des menschlichen Körpers;
- P2: Ausüben mechanischen Druckes (F) auf die Oberfläche der Vorrichtung bis ein Grenzdruck (Fₘₐₓ) erreicht ist; und
- P3: Erzeugung eines durch menschliche Sinnesorgane wahrnehmbaren erstes Signals (S3, S3');
- P4: Vollständige Rücknahme des mechanischen Druckes (F) nach Wahrnehmung des Signal (S);
- P5: Erneute Ausübung mechanischen Druckes (F) auf die Oberfläche der Vorrichtung, wobei die Schritte P2 bis P4 wiederholt werden.
- P6: Anlegen der Elektroden an den zu behandelnden Körper; und
- P7: Entladung des Energiespeichers.

Vorteilhaft ist es dabei, dass der Energiespeicher sowohl durch eine Batterie als auch einen mechanisch zu betreibenden Dynamo aufgeladen werden kann, wobei die elektronische Einheit in dem Gehäuse des Herz-Lungen-Massagegerätes integriert ist.

Weitere erfindungswesentliche Merkmale sind der Beschreibung und den Unteransprüchen zu entnehmen.

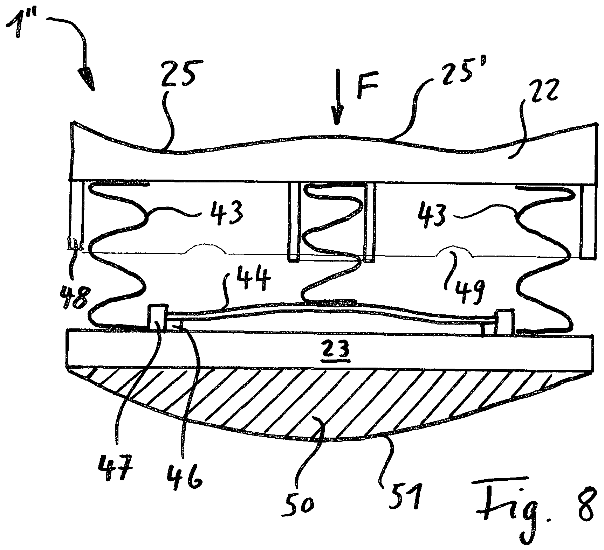

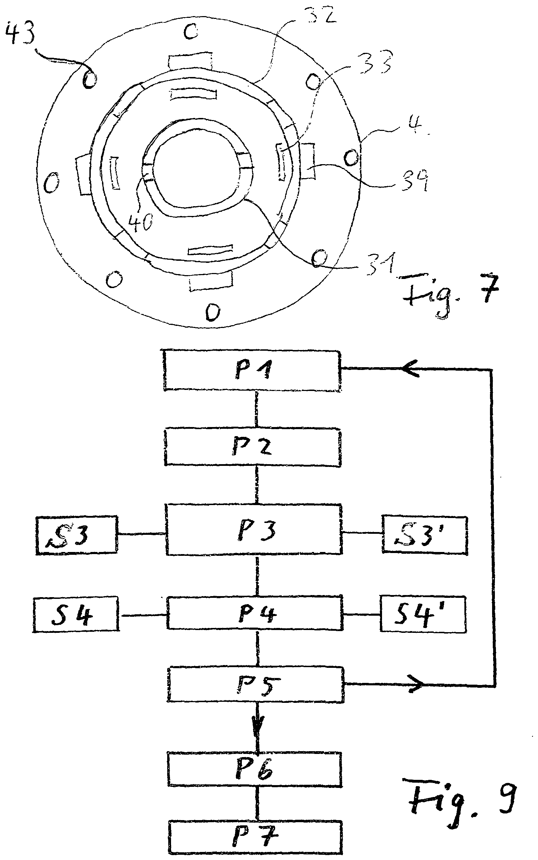

Im nun Folgenden wird die Erfindung anhand von Zeichnungen näher erläutert, wobei nur die Fign. 7, 8 Ausführungsformen zeigen, die von den Ansprüchen abgedeckt sind. Es zeigt
Figur 1 eine schematische Seitenansicht eines Beispiels einer Vorrichtung (1) mit mindestens einem ersten Druckübertragungsmittel (2) und mindestens einem starren Druck aufnehmenden Element (3);
Figur 2 eine schematische Explosionsdarstellung eines weiteren Beispiels (1') mit einem konischen Ringelement (13) und einem Druck aufnehmenden Federelement (16) (Tellerfeder);
Figur 3 eine schematische Explosions-Schnittdarstellung eines Beispiels (20) mit einem ersten Druckübertragungsmittel (22) und mindestens einem Druck aufnehmenden Element (23), sowie einem flächigen einstückigen Federelement (24);
Figur 4 einen schematischen Querschnitt durch ein trapezförmiges Federelement (24) (Tellerfeder);
Figur 5 eine schematische Draufsicht auf das flächige ringförmige Federelement (24) mit einer mittigen Öffnung (21);
Figur 6 eine schematische Draufsicht auf das Druck aufnehmende starre Element (23) mit konzentrischen Erhebungen (31, 32, 33, 35), sowie mindestens einem Durchbruch (30);
Figur 7 eine schematische Draufsicht auf das druckaufnehmende starre Element (23) mit konzentrischen Erhebungen (31, 32, 33, 35) aus Fig. 6, wobei eine Mehrzahl von Federelementen 43 auf einer Kreisbahn um das mittlere Federelement (24, 44) angeordnet sind;
Figur 8 eine schematische Seitenansicht eines Ausführungsbeispiels (1") mit einer Mehrzahl von Federelementen (43) um das mittlere flächige Federelement (24, 44) herum;
Figur 9 eine schematische Blockdarstellung der einzelnen Verfahrensschritte (PI - P7) während des Reanimationsvorgangs.

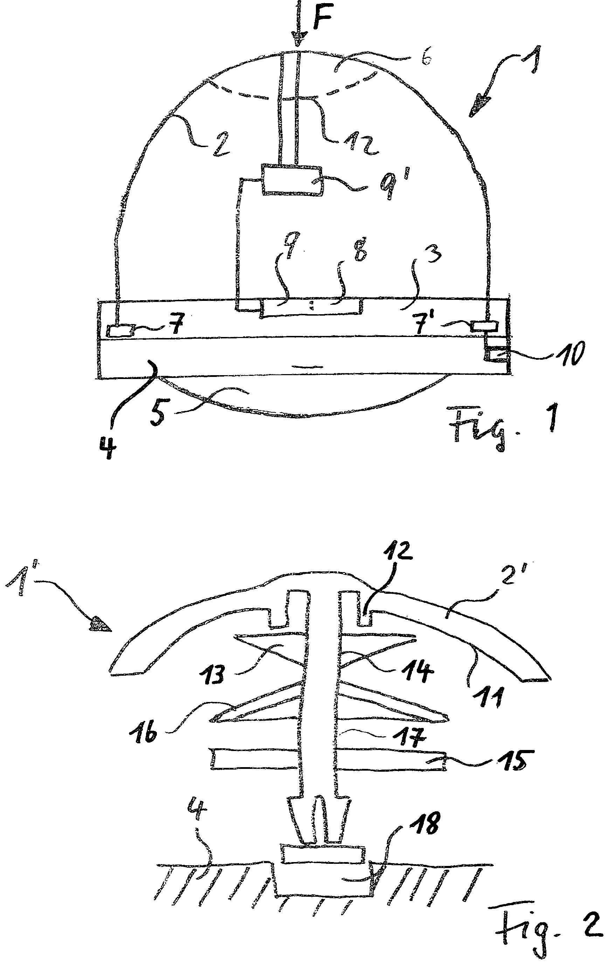

Die Figur 1 zeigt eine schematische Seitenansicht eines Beispiels der Vorrichtung 1 mit seinen wesentlichen Bauteilen. Diese Vorrichtung 1 besteht aus einem ersten Druckübertagungsmittel 2, das bogenförmig ausgebildet ist und an den Enden mit einem flachen, relativ starren Druck aufnehmenden Element 3 in Verbindung steht. Das starre Druck aufnehmende Element 3, das auch gebogen ausgebildet sein kann, weist an seiner Unterseite eine Schicht 4 auf, die aus einem nachgiebigen Material, zum Beispiel einem speziellen Schaumstoff wie widerstandsfähigem Moosgummi, gefertigt ist. An der Unterseite dieser nachgiebigen Schicht 4 ist entsprechend den anatomischen Gegebenheiten des Brustkorbs ein Formteil 5 angeordnet, dass somit eine richtige und sicherere Positionierung der Vorrichtung 1 auf dem Thorax des Brustkorbs gewährleistet. Das nachgiebige Element 4 und das Formteil 5 können auch als ein einziges Formteil aus einem geeigneten Schaumstoff, der auf der Haut des menschlichen Körpers nicht verruscht, ausgebildet sein. Das gebogene Druckübertragungsmittel 2 ist derart ausgebildet, dass bei Ausübung eines mechanischen Druckes (F) die Oberfläche des Druckübertragungsmittels 2 zunächst eine mechanische Spannung erfährt und bei Eintritt des Grenzdruckes (Fₘₐₓ) ein Teilbereich der Oberfläche sprunghaft einknickt und dabei einen deutlich wahrnehmbaren Klickton erzeugt, der dem Anwender signalisiert, dass der Maximaldruck (Fₘₐₓ) erreicht ist. Dabei dient das gewölbt ausgebildete Übertragungsmittel 2 unter anderem als Resonanzkörper, der das akustische Signal (S3) deutlich erhöht. Die Oberfläche 6 knickt dabei sprunghaft bei der vorgegebenen Druckbelastung (Fₘₐₓ) nach innen bis zu der gestrichelten Linie 12 oder ähnlich ein und erzeugt somit das deutlich hörbares Klicksignal (S3). In einer Weiterentwicklung dieses. Beispiels sind am Ende des Druckübertragungsmittels 2 Druck aufnehmende Sensoren 7, 7', z.B. Dehnungsmeßstreifen, angeordnet, die der Elektronik 8 ein Signal zuführen und dadurch ein deutlich wahrnehmbares akustisches oder optisches Signal (S3') erzeugt wird. Die beispielsweise in dem Druckübertragungsmittel 3 integrierte Elektronik wird von einem ebenfalls internen oder externen Energiespeicher 9 gespeist, der z.B. eine Batterie oder ein Kondensator ist. Der elektrische Kontakt 10 ist u.a. mit den Sensoren 7, 7' verbunden und kann auch als USB-Steckdose ausgebildet werden, um damit weitere kardiologisch wichtige Signale zu übertragen.

In einem weiteren Beispiel steht mit dem Druckübertragungsmittel 2, 6 ein Generator 9' (z. B. Dynamo) zur Energieerzeugung mechanisch in Verbindung, der durch die zyklischen Druckbewegungen der Oberfläche 2 in Bewegung gesetzt wird und dadurch Energie erzeugt, die elektrisch dem Energiespeicher 9 zugeführt wird.

In einem weiteren Beispiel ist das starre Druck aufnehmende Element 3 und das erste Kraftübertragungsmittel 2 rund, kann aber auch oval ausgebildet werden, was jedoch lediglich eine symbolische Andeutung darstellt, so dass auch jede andere beliebige sinnvolle Ausformung der einzelnen Bauelemente in Frage kommt. Wichtig ist es, dass die Form den ergonomischen Formen der Hand und des Brustkorps so angepasst ist, dass alle Druckübertragungselemente eine optimale Druckausübung und gleichmäßige Druckübertragung zunächst auf das Druckübertragungselement 2, 3 einerseits und auf dem Brustkorps andererseits bewirken. Das flache starre Kraftübertragungselement 3 ist im vorliegenden Beispiel leicht größer als der Durchmesser des Kraftübertragungselements 2, wodurch ein kleiner Rand gebildet wird. Auf diesem Rand oder an einer anderen geeigneten Stelle der Vorrichtung 1 sind zwei Markierungselemente angeordnet, die als Positionierungshilfen für das richtige Aufsetzen der Vorrichtung 1 auf den Thorax des menschlichen Körpers dienen. Eine weitere Positionierhilfe kann ein flexibles Element, z.B. ein Band oder eine Schnur, sein, das um den Hals des Patienten gelegt wird, um dadurch rasch die richtige Herzposition aufzufinden.

Die Fig 2 zeigt schematisch ein weiteres Beispiel 1' in einer Explosionsdarstellung der wichtigsten konstruktiven Bauelemente. Hierbei weist das gewölbte Druckübertragungsmittel 2' an der Unterseite 11 einen ringförmigen Steg 12 auf, der auf ein konisches Ringelement 13 mit einer Mittelbohrung 14 bei Druckerzeugung (F) einwirkt. Zwischen einer weiteren flachen Ringscheibe 15 ist mindestens ein elastisches Druck aufnehmendes Federelement 16 angeordnet, das im vorliegenden Beispiel als erhobene Tellerfeder aus Federstahl ausgebildet ist, die bei einer vorbestimmten Belastung (Fₘₐₓ) umschlägt, das heißt gegen das konische Ringelement 13 schlägt. Konische Tellerfedern aus DIN-gerechtem Federstahl in gleichsinniger und/oder wechselsinniger Anordnung eignen sich wegen ihres kleinen Federwegs bei hoher Federkraft besonders gut als Druck aufnehmendes Element 16 in der vorliegenden Erfindung. Der Schlag des Federelements überträgt sich somit über den ringförmigen Steg 12 auf das erste Druckübertragungselement 2', das als Resonanzkörper dient und bei Aufschlag der Tellerfeder 16 einen deutlich hörbaren Ton erzeugt. Der in dieser Darstellung nach unten weisende Führungsstab 17, an dem strahlenförmig, hier nicht gezeigten, Rippen angeordnet sind, dient unter anderem der zentrischen und homogenen Kraftübertragung auf das Formteil 4, bzw. 5, das mittig eine Ausnehmung 18 aufweist, in die der Führungsstab 17 bei Kraftausübung auf das erste Druckübertragungsmittel 2' eintaucht. Die Tellerfeder, bzw. das Druck aufnehmende Federelement 16, kann jede beliebige äußere Form annehmen. Die äußere Form des Federelements 16 kann rund, oval, mehreckig oder sternförmig ausgeformt sein. Wichtig ist dabei, dass sie bei einer vorbestimmten Belastung (Fₘₐₓ) ein Signal, gleich welcher Art, wie w.o. beschrieben, erzeugt.

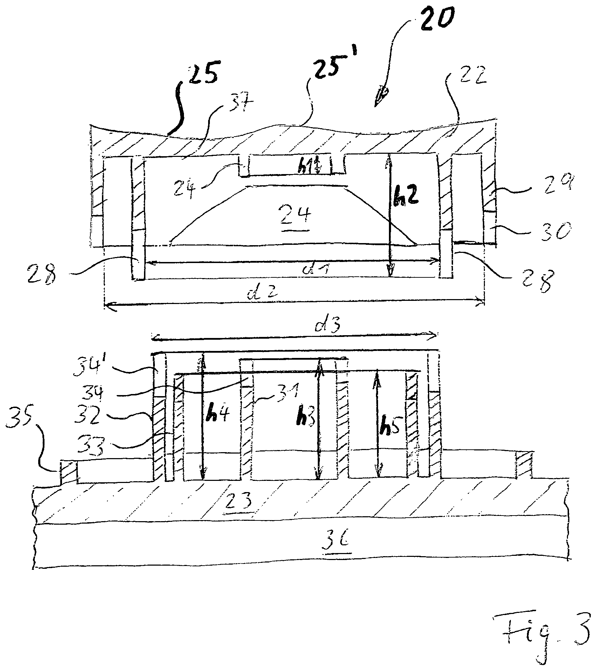

Die Fig. 3 zeigt eine schematische Schnittdarstellung eines weiteren Beispiels 20 mit einem ersten Druckübertragungsmittel 22 und mindestens einem Druck aufnehmenden Element 23, sowie einem Federelement 24, das prinzipiell zwischen dem ersten Druckübertragungsmittel 22 und dem Druck aufnehmenden Element 23 angeordnet ist. Das erste Druckübertragungsmittel 22 ist im Schnitt etwa u-förmig ausgebildet und weist an seiner Oberseite eine Deckfläche 25 auf, die konkave ausgebildet ist und mittig eine kleine Erhöhung 25' oder Wölbung aufweisen kann, die ergonomischen und Zentrierzwecken dient. Das erste Druckübertragungsmittel 22 ist im vorliegenden

Beispiel rund ausgebildet, was aber nicht zwingend ist. Ebenso sind ovale, herzförmige oder mehreckige Ausführungsformen denkbar. Auf der Innenseite 26 des Druckübertragungsmittels 22 ist ein konzentrischer Ringsteg 27 angeordnet, dessen Durchmesser etwa dem Durchmesser g2 der Öffnung des Federelements 24 entspricht. Dieser Ringsteg 27 ist in seiner Höhe h1 im Vergleich zur Höhe h2 einer weiteren ringförmigen Erhebung 28 innerhalb des ersten Druckübertragungsmittels 22 vergleichsweise klein. Die Durchmesser der Erhebungen 27 und 28 nehmen etwa die Größe der Durchmesser g1 und g2 des Federelements 24 an und muss so groß sein, dass die ringförmige Erhebung 28 über eine weitere, unten zu beschreibende ringförmige Erhebung 32 passt. Die Seitenwände 29 sind ebenfalls ringförmig ausgebildet und weisen eine Mehrzahl von Ausnehmungen 30 auf. Das Federelement 24 wird weiter unten näher beschrieben. Unterhalb des ersten Druckübertragungsmittels 22 ist ein relativ starres Druck aufnehmendes Element 23 angeordnet, das als Basisplatte angesehen werden kann, auf der mehrere ringförmige Erhebungen 31, 32, 33 ausgebildet sind. Die mittlere ringförmige Erhebung 31 ist in ihrer Höhe h3 größer als die Höhe h1 der ringförmigen gegenüber liegenden Erhebung 27 im ersten Druckübertragungsmittel 22, was mit der Geometrie des Federelements 24 zusammen hängt. Konzentrisch zu der ringförmigen Erhebung 31 ist eine weitere ringförmige Erhebung 32 angeordnet, die in ihrer Höhe h4 geringfügig höher ist als die mittlere ringförmige Erhebung 31. Der Durchmesser der ringförmigen Erhebung 31 entspricht in etwa dem Durchmesser g2 der ringförmigen Erhebung 27 innerhalb des ersten Druckübertragungsmittels 22 und dient u.a. als Führungsmittel. In unmittelbarer Nähe der ringförmigen Erhebung 32 sind isoliert mehrere Federstützelemente 33 angeordnet, auf die das Federelement 24 lose aufgelegt wird. Die Höhe h5 dieser Federstützelemente 33 liegt etwas unter der Höhe h3 der ringförmigen Erhöhung 31, damit der Aufschlag des Federelements 24 bei Erreichen des Grenzdruckes (Fₘₐₓ) kräftig auf die mittele Erhöhung 31 aufschlägt und bei Nachlassen des mechanischen Druckes (F) das Federelement 24 sofort wieder zurück in die Ausgangsposition springt. Die ringförmigen Erhebungen 31, 32, 35 weisen jeweils mindestens eine Ausnehmung 34, 34' auf, die auf dem gesamten Umfang der Erhebungen verteilt sind. Konzentrisch zu den beiden Erhebungen 31, 32 ist eine weitere ringförmige Erhebung 35 angeordnet, deren Durchmesser d2 kleiner ist als der Innendurchmesser d1 des ersten Druckübertragungsmittels 22. Unterhalb der Basisplatte 23 ist ein elastisches Material als Druckübertragungsmittel 36 angeordnet, das direkt mit dem Brustkorb des zu behandelnden Körpers in Berührung steht. Das Material ist ein spezieller Schaumstoff, z. B. ein Moosgummi, wie weiter oben beschrieben wurde.

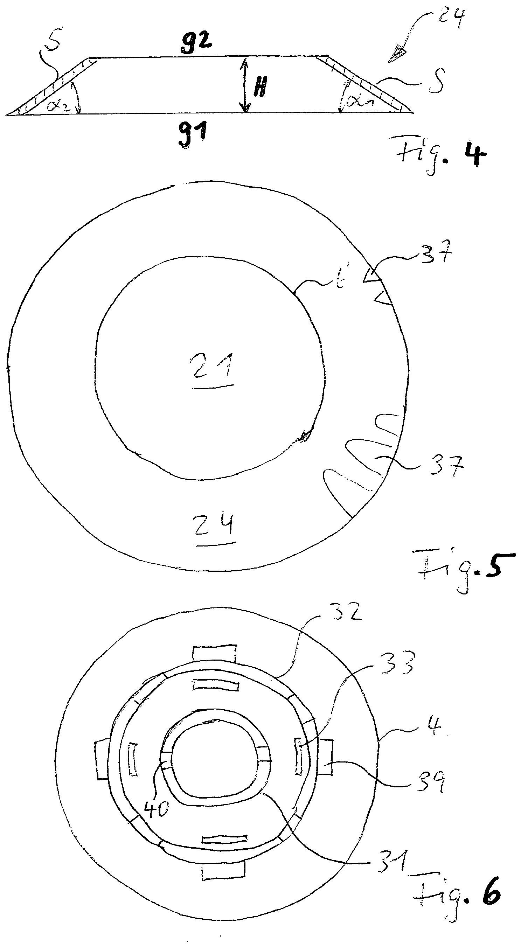

Die Fig. 4 zeigt einen schematischen Querschnitt durch ein trapezförmig ausgebildetes Federelement 24, das zwischen dem ersten Druckübertragungsmittel 22 und dem Druck aufnehmenden Element 23 (Basisplatte) angeordnet ist. Das Federelement 24 ist im vorliegenden Beispiel ringscheibenförmig rund ausgebildet, wobei die flächigen Seiten (S) des Trapezes leicht gewölbt sein können und die Winkel (α1) und (α2), die von der Grundlinie g1 und den Seiten (s) eingeschlossen sind, nicht notwendig gleich sein müssen. Die Grundlinie g2 stellt praktisch die Öffnung der Ringscheibe dar. Die Höhe H der trapezförmigen Tellerscheibe liegt im vorliegenden Beispiel zwischen 1,3 mm und 2,9 mm, wobei die Wandstärke des Federelements 24 ca. 0,3 mm beträgt und der Außendurchmesser (D) zwischen 44 mm und 55 mm liegt.

Die Fig. 5 zeigt die Draufsicht auf das ringförmige Federelement 24 aus Fig. 4. Der Innendurchmesser der Ringscheibe liegt zwischen 7 mm und 32 mm. Am Umfang des Außendurchmessers (g1) ist eine Mehrzahl von Verformungen 37 angeordnet, die verschiedene Ausprägungen haben können, z.B. wellenförmig und/oder u-förmig. Ferner können im Umfangsbereich Ausnehmungen 38 angeordnet werden, die beide u.a. auf die Steifigkeit des Federelements 24 einwirken. Andere Deformationen, wie z.B. Bohrungen, sind ebenfalls zu diesem Zwecke möglich.

Die Fig. 6 zeigt ausschnittsweise eine schematische Draufsicht auf das Druck aufnehmenden Teil 23 (Basisplatte) der Vorrichtung 1'. Wie bereits beschrieben, nimmt die Basisplatte 23 konzentrisch angeordnete Erhebungen auf, wovon die kreisringförmigen Erhebungen unterschiedliche Höhen h1, h2, h3, h4, h5 aufweisen. Die Basisplatte 23 selbst kann eine ebene oder geschwungene Fläche darstellen, die mindestens eine Ausnehmung aufweist, wobei die möglichen Durchbrüche 39 im Allgemeinen diametral zueinander angeordnet sind, und dienen u.a. zum Eingriff für einen Befestigungshaken, der an der Erhebung 28 im Innern des ersten Druckübertragungsmittels 22 angeordnet ist, die über die kreisringförmige Erhebung 32 auf der Basisplatte 23 geschoben wird. Die kreisringförmige Erhebung 31 weist eine Mehrzahl von Ausnehmungen 34, verteilt über den gesamten Umfang, auf. Innerhalb des kreisringförmigen Erhebung 32, in der Umgebung der Durchbrüche 39 sind isoliert von der kreisringförmigen Erhebung 32 Stützelemente 33 zur Auflage des Federelements 24 diametral zu einander angeordnet, die eine Höhe h5 aufweisen. Die mittlere kreisringförmige Erhebung 31 weist im vorliegenden

Beispiel relativ schmale Einkerbungen 40 auf, die sich gegenüberliegen. Die äußere kreisringförmige Erhebung 41 dient der Abgrenzung der Basisplatte 23 und zur Aufnahme der Seitenwände 29 des ersten Druckübertragungsmittels 22, dessen Durchmesser etwa zwischen 70 mm und 120 mm liegt. Die gesamte Basisplatte 23 ist eher oval oder tropfenförmig mit einer Längsausdehnung von ca. 120 mm ausgebildet.

Die Fig. 7 zeigt eine schematische Draufsicht auf das Druck aufnehmende starre Element 23 mit konzentrischen Erhebungen (31, 32, 33, 35) aus Fig. 6, wobei eine Mehrzahl von Federelementen 43 auf einer Kreisbahn konzentrisch angeordnet sind und mit dem oberen Druckübertragungsmittel 2, 22 in Verbindung steht, um den mechanischen Druck (F), der auf das erste Druckübertragungsmittel ausgeübt wird, zu konzentrieren und auf das mindestens eine flächige Federelement 24 zu übertragen. Damit ist das erste Druckübertragungsmittel 22 praktisch schwimmend abgefedert.

Die Fig. 8 zeigt eine schematische Seitenansicht eines weiteren Ausführungsbeispiels der erfindungsgemäßen Vorrichtung 1" zur Herz-Lungen-Reanimation eines menschlichen Körpers mit einer Mehrzahl von Federelementen 43 um das mittlere Federelement 44 herum, über dem ein weiteres Federelement 45 angeordnet ist, das mit seinem einen Ende die Oberfläche des flächigen Federelements 44 berührt. Die Federelemente 43 sind zwischen dem ersten Druckübertragungsmittel 2, 22 und dem Druck aufnehmenden Element 3 angeordnet, wobei das eine Ende des Federelementes 43 die Unterseite des ersten Druckübertagungsmittels 2, 22 berührt und das andere Ende die Innenseite des Druck aufnehmenden Elements 23. Die mindestens zwei Federelemente 43 sind im vorliegenden Ausführungsbeispiel als Wendel-Druck-Federn ausgebildet, die den mechanischen Druck (F) vom ersten Druckübertragungsmittel 22 auf das Druck aufnehmende Element 23 übertragen. Gleichzeitig wird der Druck des ersten Druckübertragungsmittels 2' mittels des weiteren Federelementes 45 auf das flächige Federelement 44 übertragen, wodurch das flächige Federelement 44 bei Kraftausübung auf das erste Druckübertragungsmittel 22 nach unten durchgebogen wird und bei einer vorbestimmten Durchbiegung spontan in eine Enddurchbiegung springt und dabei ein deutlich wahrnehmbares Signal (S3) erzeugt. Bei Nachlassen des mechanischen Druckes (Fₘₐₓ) auf das erste Druckübertragungsmittel 22 springt das flächige Federelement 44 wieder zurück in die Ausgangsposition und schlägt dabei mit der Oberfläche an das untere Ende des Federelementes 45, wodurch ein zweites deutlich wahrnehmbares Signal (S4) erzeugt wird. Das weitere Federelement 45 sitzt in einer Führung 46, die gleichzeitig als Resonanzkörper im Zusammenwirken mit den Federelementen 44, 45 dient. Die Federkonstanten der Federelemente 43 (Wendelfedern) sind so gewählt, dass das erste wahrnehmbare Signal (S3) bei einer einstellbaren mechanische Kraft zwischen 40 kg und 50 kg erzeugt wird und das zweite wahrnehmbare Signal (S4) bei Unterschreitung der eingestellten Kraft (F), die auf das Kraftübertragungsmittel 22 einwirkt. Die Einstellung der mechanischen Kraft (Fₘₐₓ) erfolgt durch das Zusammenwirken aller beteiligten Federelemente. Das flächige Federelement 44 liegt frei auf einer Mehrzahl von Auflageelementen 46 auf, wobei der Rand des flächigen Federelementes 44 durch seitliche Stützen 47 abgestützt wird. Die nach oben weisende Oberfläche 25 des ersten Kraftübertragungsmittels 22 ist im Wesentlichen konkave ausgebildet, wobei für bestimmte Anwendungsfälle die Oberfläche mittig eine leichte Ballung 25' aufweist. Die besondere schalenförmige Ausbildung der Oberfläche ist für eine dezentrale Kraftausübung während der Behandlung des Patienten von Bedeutung. Die Federelemente 43 (Wendelfedern) sind von einer seitlichen Begrenzung 48 umgeben, die am unteren Rand eine Mehrzahl von (bogenförmigen) Ausnehmungen 49 aufweist. Unterhalb des Kraft aufnehmenden Elementes 23 ist mindestens eine Schicht 50 eines nachgiebigen flexiblen Materials, z. B Schaumstoff, angeordnet, dessen nach außen weisende Oberfläche 51 im Wesentlichen konvex ausgebildet ist, um sich günstig an die anatomischen Gegebenheiten des Brustkorpus anzupassen.

Die Fig. 9 zeigt eine Blockdarstellung der wesentlichen Verfahrensschritte (P1 - P7) bei Anwendung der erfindungsgemäßen Vorrichtung 1. Der erste Schritt P1 besteht darin, die Vorrichtung wirksam auf dem Brustkorb an vorbestimmter Stelle aufzusetzen und zu positionieren. Der zweite Schritt P2 besteht darin, mit der Hand einen mechanischen Druck (F) auf die Oberfläche 24, 25 des Druckübertragungsmittels 2, 2' 22 der Vorrichtung 1, 1', 1", 20 auszuüben, bis ein Grenzdruck (Fₘₐₓ) erreicht ist, wobei der Grenzdruck etwa zwischen 40 und 50 kg liegt, vorzugsweise jedoch bei 45 kg zur Anwendung kommt. Nach Erreichen des Grenzdruckes (Fₘₐₓ) wird von der Vorrichtung 1 ein von einem menschlichen Sinnesorgan wahrnehmbares Signal (S3, S3'), entweder mechanisch und/oder elektrisch erzeugt, das dem Anwender sagt, dass der Grenzdruck (Fₘₐₓ) erreicht ist. Sobald dieser Grenzdruck erreicht wurde, ist der mechanische Druck sofort vollständig zurückzunehmen (P4), wodurch ein zweites Signal (S4, S4') erzeugt wird. Nach einer kurzen vorbestimmten Zeit ist dann in einem weiteren Verfahrensschritt (P5) der Druckvorgang zyklisch zu wiederholen.

In einem Ausführungsbeispiel der vorliegenden Erfindung ist, wie weiter oben beschrieben wurde, eine integrierte Elektronik 9 vorgesehen, die der Erzeugung eines Strom-Spannungsimpulses dient, so dass nach erfolgter Herz-Lungenmassage (P1- P5) die Schritte P6 - P7 folgen, wobei P6 das Anlegen der Elektroden des Defibrillators und P7 die Entladung mittels eines bestimmten Signals des entsprechenden Energiespeichers bedeutet.

## Patentansprüche

1. Vorrichtung (1) zur kontrollierten Herz-Lungen-Reanimation des menschlichen Körpers bei Herzstillstand, mit mindestens einem Druckübertragungsmittel (22) und mindestens einem Druck aufnehmenden Element (23) und einer Druckanzeige, die bei Eintritt eines einstellbaren Grenzdruckes (Fₘₐₓ) ein durch menschliche Sinnesorgane wahrnehmbares Signal (S) erzeugt, wobei Vorrichtung mindestens ein Federsystem mit einer Mehrzahl von Federelementen (43, 44, 45) umfasst, die bei Ausübung eines mechanischen Druckes (F) zusammenwirken und die Einstellung des Grenzdruckes durch Zusammenwirken aller Federelemente erfolgt, wobei mindestens ein Federelement (44) des Federsystems flächig und einstückig ausgebildet ist, **dadurch gekennzeichne**t, dass mindestens zwei Federelemente (43) des Federsystems seitlich zum flächigen einstückigen Federelement (44) und um das flächige einstückige Federelement (44) herum angeordnet sind, wobei oberhalb des mindestens einen flächigen einstückigen Federelements (44) mindestens ein weiteres Federelement (45) des Federsystems angeordnet ist, das den mechanischen Druck auf das mindestens eine Druckübertragungsmittel (22) aufnimmt und auf das flächige einstückige Federelement (44) überträgt.

2. Vorrichtung nach Anspruch 1, **dadurch gekennzeichnet, dass** bei Erreichen des einstellbaren Grenzdruckes (Fₘₐₓ) als auch beim Zurückspringen in die Startposition ein deutlich hörbares Signal (S) erzeugt wird, das sowohl mechanisch als auch akustisch auf die das flächige einstückige Federelement (4) tragenden Elemente einwirkt.

3. Vorrichtung nach Anspruch 1, **dadurch gekennzeichnet, dass** das flächige einstückige Federelement (44) ringscheibenförmig ausgebildet ist.

4. Vorrichtung nach Anspruch 1, **dadurch gekennzeichnet, dass** die Randbereiche des Innen- und Außenumfangs des als Tellerfeder (44) ausgebildeten flächigen einstückigen Federelements wellenförmige und /oder mehreckige Deformationen (37) und Ausnehmungen (38) und/oder Bohrungen aufweisen, die die Steifigkeit des Federelements beeinflussen und definieren.

5. Vorrichtung nach Anspruch 1, **dadurch gekennzeichnet, dass** der Außendurchmesser (g1) des flächigen einstückigen Federelements (44) zwischen ca. 30 mm und ca.75 mm, vorzugsweise bei 47 mm liegen kann.

6. Vorrichtung nach Anspruch 1, **dadurch gekennzeichnet, dass** auf einer ebenen oder gewölbten Basisfläche (23) mit mindestens einem Durchbruch (39) konzentrisch Erhebungen (32, 33, 34, 35) angeordnet sind, die mittig das flächige einstückige Federelement (44) aufnehmen, wobei die Erhebungen kreisförmig, mehreckig oder wellenförmig als Ringe mit mindestens einer Ausnehmung (34, 34') angeordnet sein können.

7. Vorrichtung nach Anspruch 1, **dadurch gekennzeichnet, dass** mindestens ein Federstützelement (33) isoliert von seiner Nachbarerhebung (32) angeordnet ist.

8. Vorrichtung nach einem der vorangegangenen Ansprüche, **dadurch gekennzeichnet, dass** auf der Außenseite des Druck aufnehmenden Elements (23) ein Schaumstoff (36) angeordnet ist, dessen Oberfläche ballig ausgebildet ist.

9. Vorrichtung nach Anspruch 1, **dadurch gekennzeichnet, dass** die Deckfläche (25) des zylinderförmig ausgebildeten mindestens einen Druckübertragungsmittels (22) konkav ausgebildet ist, wobei die Deckfläche mindestens eine Wölbung (25') aufweist und die Seitenwand (29) des zylinderförmig ausgebildeten Druckübertragungsmittels (22) mindestens eine Ausnehmung (30), vorzugsweise eine Mehrzahl von Ausnehmungen, aufweist.

10. Vorrichtung nach einem der vorangegangenen Ansprüche, **dadurch gekennzeichnet, dass** das mindestens eine flächige einstückige Federelement (44) rückexpandierend ausgebildet ist und dabei mindestens ein zweites Signal (S4) erzeugt.

## Claims

1. Device (1) for controlled cardiopulmonary resuscitation of the human body in cardiac arrest, with at least one pressure transmission means (22) and at least one pressure-absorbing element (23) and a pressure display, which occurs when an adjustable limit pressure (Fmax) occurs human sensory organs generates a perceptible signal (S), whereas the device comprising at least one spring system with a plurality of spring elements (43, 44, 45), which interact when mechanical pressure (F) is exerted and the limit pressure is set by interaction of all spring elements, wherein at least one spring element (44) of the spring system is flat and in one piece, **characterized in that** at least two spring elements (43) of the spring system are arranged laterally to the flat one-piece spring element (44) and around the flat one-piece spring element (44), and above the at least one area A one-piece spring element (44) is arranged at least one further spring element (45) of the spring system, which absorbs the mechanical pressure on the at least one pressure transmission means (22) and transfers it to the flat one-piece spring element (44).

2. Device according to claim 1, **characterized in that** when the adjustable limit pressure (Fmax) is reached as well as when jumping back into the starting position, a clearly audible signal (S) is generated which is both mechanically and acoustically based on the flat, one-piece spring element (44) supporting elements.

3. Device according to claim 1, **characterized in that** the flat one-piece spring element (44) is annular.

4. Device according to claim 1, **characterized in that** the edge areas of the inner and outer circumference of the flat one-piece spring element designed as a plate spring (44) have wavy and / or polygonal deformations (37) and recesses (38) and / or bores which influence and define the rigidity of the spring element.

5. Device according to claim 1, **characterized in that** the outer diameter (g1) of the flat, one-piece spring element (44) can be between approximately 30 mm and approximately 75 mm, preferably 47 mm.

6. Device according to claim 1, **characterized in that** on a flat or curved base surface (23) with at least one opening (39) concentric elevations (32, 33, 34, 35) are arranged, which receive the flat one-piece spring element (44) in the center, the elevations being circular, polygonal or wavy as rings with at least one recess (34, 34').

7. Device according to claim 1, **characterized in that** at least one spring support element (33) is arranged isolated from its neighboring elevation (32).

8. Device according to one of the preceding claims, **characterized in that** a foam (36) is arranged on the outside of the pressure-absorbing element (23), the surface of which is spherical.

9. Device according to claim 1, **characterized in that** the top surface (25) of the cylinder-shaped at least one pressure transmission means (42) is concave, the top surface having at least one curvature (25 ') and the side wall (29) of the cylinder-shaped pressure transmission means (22) has at least one recess (30), preferably a plurality of recesses.

10. Device according to one of the preceding claims, **characterized in that** the at least one flat one-piece spring element (44) is designed to expand back and thereby generates at least one second signal (S4).

## Revendications

1. Dispositif (1) de réanimation cardio-respiratoire contrôlée du corps humain en arrêt cardiaque, avec au moins un moyen de transmission de pression (22) et au moins un élément absorbeur de pression (23) et un affichage de pression, qui se produit lorsqu'une pression limite réglable (Fmax) se produit les organes sensoriels humains génèrent un signal perceptible (S), le dispositif comprenant au moins un système à ressort avec une pluralité d'éléments à ressort (43, 44, 45), qui interagissent lorsque la pression mécanique (F) est exercée et la pression limite est fixée par interaction de tous les éléments à ressort, dans lequel au moins un élément de ressort (44) du système de ressort est plat et monobloc, **caractérisé en ce qu**'au moins deux éléments de ressort (43) du système de ressort sont disposés latéralement par rapport à l'élément de ressort monobloc plat (44) et autour de l'élément de ressort monobloc plat (44), dans lequel au moins un autre élément de ressort (45) du système de ressort est disposé au-dessus de l'au moins un élément de ressort plat et monobloc (44) du système de ressort, qui absorbe la pression mécanique sur le au moins un moyen de transmission de pression (22) et la transfère à l'élément ressort plat monobloc (44).

2. Dispositif selon la revendication 1, **caractérisé en ce que** lorsque la pression limite réglable (Fmax) est atteinte ainsi que lors du saut dans la position de départ, un signal clairement audible (S) est généré qui est à la fois mécaniquement et acoustiquement basé sur l'élément de ressort plat monobloc (44) éléments de support.

3. Appareil selon la revendication 1, **caractérisé en ce que** le l'élément de ressort monobloc plat (44) est annulaire.

4. Appareil selon la revendication 1, **caractérisé en ce que** les zones de bord de la circonférence intérieure et extérieure de l'élément ressort monobloc plat conçu comme un ressort à lame (44) présentent des déformations ondulées et / ou polygonales (37) et des évidements (38) et / ou des alésages qui influencent et définissent la rigidité de l'élément ressort.

5. Dispositif selon la revendication 1, **caractérisé en ce que** le diamètre extérieur (g1) de l'élément ressort plat monobloc (44) peut être compris entre environ 30 mm et environ 75 mm, de préférence 47 mm.

6. Dispositif selon la revendication 1, **caractérisé en ce que** sur une surface de base plate ou incurvée (23) avec au moins une ouverture (39) des élévations concentriques (32, 33, 34, 35) sont disposées, qui reçoivent l'élément de ressort plat monobloc (44) au centre, les élévations étant circulaires, polygonales ou ondulées sous forme d'anneaux avec au moins un évidement (34, 34').

7. Dispositif selon la revendication 1, **caractérisé en ce qu'**au moins un élément de support de ressort (33) est disposé isolé de son élévation voisine (32).

8. Dispositif selon l'une des revendications précédentes, **caractérisé en ce qu**'une mousse (36) est disposée à l'extérieur de l'élément absorbant la pression (23), dont la surface est sphérique.

9. Dispositif selon la revendication 1, **caractérisé en ce que** la surface supérieure (25) du moyen de transmission de pression en forme de cylindre (42) est concave, la surface supérieure ayant au moins une courbure (25 ') et la paroi latérale (29) du moyen de transmission de pression en forme de cylindre (22) possède au moins un évidement (30), de préférence une pluralité d'évidements.

10. Dispositif selon l'une des revendications précédentes, **caractérisé en ce que** l'au moins un élément ressort plat monobloc (44) est conçu pour se dilater en arrière et ainsi générer au moins un second signal (S4).
